# EUROPÄISCHE PATENTANMELDUNG

(11) **EP 2 060 277 A1**
(43) Veröffentlichungstag der Anmeldung: **20.05.2009**
(21) Anmeldenummer: 07022144.5
(22) Anmeldetag: 14.11.2007
(51) Int. Cl.: A61L 2/20, A61L 2/22, A61L 2/24, B65B 55/02, B67C 7/00

(54) **Vorrichtung und Verfahren zum Sterilisieren**

(71) Anmelder: INDAG Gesellschaft für Industriebedarf mbH & Co. Betriebs KG, 69214 Eppelheim (DE)
(72) Erfinder: Wild, Hans-Peter, 69214 Eppelheim (DE); Tilz, Wolfgang, 68723 Schwetzingen (DE)
(74) Vertreter: Grünecker, Kinkeldey, Stockmair & Schwanhäusser Anwaltssozietät

(57) **Zusammenfassung**

Die Erfindung betrifft eine Vorrichtung und ein Verfahren zum Sterilisieren, insbesondere zum Sterilisieren eines Anschlussbereichs eines Lebensmitteltanks mit einem Sterilisationskopf, in dem eine Einspritzdüse für H₂O₂ angeordnet ist. Um zu bestimmen, ob eine Sterilisation stattgefunden hat, ist in dem Sterilisationskopf ein Sensor angeordnet, der die Sauerstoffkonzentration in dem Sterilisationskopf erfasst.

## Beschreibung

Die Erfindung betrifft eine Vorrichtung und ein Verfahren zum Sterilisieren, insbesondere zum Sterilisieren eines Anschlussbereichs eines Lebensmitteltanks, gemäß den Oberbegriffen der Ansprüche 1, 9 und 16.

Die vorliegende Erfindung eignet sich insbesondere zum Sterilisieren eines Bag-in-Tank-Systems.

Bag-in-Tank-Systeme werden zum Transport von Lebensmittelprodukten, insbesondere von flüssigen bzw. fließenden Lebensmittelprodukten, wie Getränke oder Fruchtzubereitungen, benutzt. Gegenüber den herkömmlichen Transporttanks haben diese Systeme den Vorteil, dass nach der Benutzung der Tank nicht aufwendig gereinigt werden muss, sondern nur ein neuer Inliner eingefügt werden muss. Zwar erfüllen die Innenseiten der Inliner üblicherweise die strengen Hygieneanforderungen, die bei den oben genannten Produktfamilien eingehalten werden müssen, um eine möglichst keimfreie Verpackung zu garantieren ist es jedoch auch nötig, neben dem sterilen Innenraum des Inliners auch den Anschlussbereich des Bag-in-Tank-Systems, über die der Inliner befüllt, bzw. später wieder entleert wird, entsprechend zu reinigen und zu entkeimen.

Auch für Lebensmitteltanks ohne Inliner kann es notwendig sein, den Anschlussbereich zu sterilisieren.

Bei der Sterilisation wird neben heißem Wasserdampf auch H₂O₂ verwendet.

Da die Sterilisation über einen Sterilisationskopf erfolgt, kann von außen nicht genau erkannt werden, ob tatsächlich eine ausreichende Sterilisation über Wasserstoffperoxyd stattgefunden hat. Es wäre vorteilhaft eine solche Sterilisation zu erfassen und zu dokumentieren. Allerdings ist es bislang nicht möglich gewesen auf einfache Weise zu bestimmen, ob eine H₂O₂-Sterilisation stattgefunden hat.

Hiervon ausgehend liegt der vorliegenden Erfindung die Aufgabe zugrunde, eine Vorrichtung und ein Verfahren bereitzustellen, die erfassen können, ob eine Sterilisation mittels H₂O₂ stattgefunden hat.

Erfindungsgemäß wird diese Aufgabe durch die Merkmale der Ansprüche 1, 9, sowie 16 gelöst.

Zur Erfassung, ob eine Sterilisation mittels H₂O₂ stattgefunden hat, umfasst der Sterilisationskopf einen Sensor der die Sauerstoffkonzentration in dem Sterilisationskopf erfasst. Da H₂O₂ zu H₂O und O₂ zerfällt, kann indirekt über den Anstieg der Sauerstoffkonzentration eine Aussage darüber gemacht werden, ob Wasserstoffperoxyd eingespritzt wurde oder nicht. Somit kann auf einfache Art und Weise dokumentiert werden, ob eine Sterilisation stattgefunden hat und ob diese in ausreichendem Maße stattgefunden hat.

Gemäß einer bevorzugten Ausführungsform arbeitet der Sensor nach dem Prinzip einer λ-Sonde zur Bestimmung der Sauerstoffkonzentration. Dabei kann der Sensor beispielsweise eine Nernstsonde oder aber eine Widerstandssonde umfassen. Die zuvor genannten Sonden können kostengünstig in dem Messkopf integriert werden.

Vorzugsweise umfasst die erfindungsgemäß Vorrichtung eine Auswerteeinheit, die die gemessene Sauerstoffkonzentration mit einem Schwellwert vergleicht und bei Überschreiten des Schwellwerts bestimmt, dass eine ausreichende Sterilisation mittels H₂O₂ stattgefunden hat.

Gemäß einem bevorzugten Ausführungsbeispiel ist im Sterilisationskopf weiter eine Entlüftungsleitung angeordnet und zwischen Einspritzdüse und der Entlüftungsleitung, bzw. dem offenen Ende der Entlüftungsleitung, eine Dampfbarriere angeordnet, die das Abströmen von H₂O und O₂ bei der H₂O₂-Sterilisation bremst, wobei der Sensor in einem Raum zwischen Dampfbarriere und Einspritzdüse angeordnet ist. Gemäß einer bevorzugten Ausführungsform umfasst der Messkopf auch eine Zuleitung für Dampf, die bei der Sterilisation mit H₂O₂ als Entlüftungsleitung dient.

Dadurch, dass die Dampfbarriere zwischen der Entlüftungsleitung und der Einspritzdüse vorgesehen ist, kann das bei der Sterilisation zerfallende H₂O₂ in Form von H₂O und O₂ nicht so schnell über die Entlüftungsleitung abweichen, so dass sich in dem Raum zwischen Dampfbarriere und Einspritzdüse Sauerstoff anreichern kann. Dies beschleunigt die Messung des Sauerstoffs durch den Sensor. Somit kann beispielsweise bereits nach etwa 2 bis 3 Sekunden ein deutlicher Anstieg der Sauerstoffkonzentration ermittelt werden, was die Messgenauigkeit und auch die Messdauer erheblich verbessert.

Wenn bei der Sterilisation mit H₂O₂ die Zuleitung für Dampf als Entlüftungsleitung verwendet wird, kann der Messkopf baulich stark vereinfacht werden.

Gemäß einer bevorzugten Ausführungsform weist der Messkopf ein Innenrohr auf, sowie ein darüber angeordnetes Außenrohr, wobei zwischen Innen- und Außenrohr ein Ringkanal vorgesehen ist, der die Zuleitung für Dampf darstellt, wobei die Einspritzdüse in dem Innenrohr angeordnet ist. Eine solche Anordnung ist kompakt und einfach zu bewerkstelligen.

Die Dampfbarriere kann aus einer Packung mit einer Vielzahl von Durchgangskanälen gebildet sein, die einen Druckabfall des durchströmenden Mediums bewirkt. Solche Packungen können beispielsweise aus einer Packung für Laborkolonnen (z.B. Sulzerpack), Stahlwolle, einer grobporösen Keramik etc. gebildet werden. Durch den Druckabfall des durchströmenden Mediums ergibt sich der zuvor genannte Druckanstieg in dem Raum zwischen Dampfbarriere und Einspritzdüse.

Bei dem erfindungsgemäßen Verfahren wird der Sterilisationskopf an einen Anschlussbereich angeschlossen und H₂O₂ aus der Einspritzdüse im Sterilisationskopf in Richtung Anschlussbereich eingespritzt. Dann wird die Sauerstoffkonzentration im Sterilisationskopf gemessen, nachdem das H₂O ausgestoßen wurde. Schließlich kann dann in Abhängigkeit der gemessenen Sauerstoffkonzentration bestimmt werden, ob eine Sterilisation stattgefunden hat oder aber nicht.

Vor dem Einspritzen des Wasserstoffperoxyds kann über die Zuleitung für Dampf, insbesondere Heißwasserdampf, ausgestoßen werden. Dies bringt den Vorteil mit sich, dass sich die Wände des Sterilisationskopfes aufheizen. Die Temperatur des Sterilisationskopfes, d.h. der Innenwände, betragen dann beim Ausstoßen des Wasserstoffperoxyds zwischen 90 und 130 °C, was den Zerfall von H₂O₂ begünstigt. Die Erhöhung der Temperatur kann auch durch eine Heizung erreicht werden.

Gemäß dem erfindungsgemäßen Verfahren wird die Sauerstoffkonzentration, die in einem bestimmten Zeitraum nach dem Einspritzen des H₂O₂ gemessen wurde, mit einem Schwellwert verglichen und bei Überschreiten des Schwellwerts wird bestimmt, dass eine ausreichende Sterilisation mittels H₂O₂ stattgefunden hat.

Zwischen der Dampfbarriere und der Einspritzdüse kann sich ein Druck von 10 mbar bis 100 mbar aufbauen, nachdem H₂O₂ ausgestoßen wurde.

Gemäß der Erfindung wird in einem Zeitraum von 1 bis 10 Sekunden nach dem Ausstoßen des H₂O₂ eine erhöhte Sauerstoffkonzentration gemessen.

Die vorliegende Erfindung stellt ein Verfahren bereit zum Bestimmen von H₂O₂, insbesondere zum Bestimmen ob eine Sterilisation mit H₂O₂ erfolgt ist, wobei die Sauerstoffkonzentration in einem Raum, in den H₂O₂ eingebracht wurde, mit Hilfe eines Sensors ermittelt wird. Vorteilhafterweise arbeitet dieser Sensor nach dem Prinzip einer λ-Sonde und umfasst vorzugsweise eine Nernstsonde oder eine Widerstandssonde.

Die vorliegende Erfindung wird nachfolgend unter Bezugnahme der folgenden Figuren näher erläutert.
- Fig. 1: ist ein Längsschnitt durch einen Messkopf, der an einen Anschlussbereich angeschlossen ist, gemäß der vorliegenden Erfindung.
- Fig. 2a: zeigt einen Längsschnitt durch das Innenrohr 2, das in Fig. 1 gezeigt ist.
- Fig. 2b: zeigt einen Abdeckring, der auf die Rückseite des Innenrohrs 2 aufgesetzt ist.
- Fig. 3: zeigt einen Längsschnitt durch ein Außenrohr 3.
- Fig. 4a bis 4e: zeigen den Ablauf eines Sterilisationsverfahrens gemäß der vorliegenden Erfindung.
- Fig. 5: zeigt ein Diagramm, das den gemessenen Sauerstoffgehalt in Abhängigkeit der Zeit zeigt.
- Fig. 6: zeigt ein Bag-in-Tank-System.
- Fig. 7: zeigt ein Ablaufdiagramm für ein erfindungsgemäßes Verfahren.
- Fig. 8: erläutert ein Funktionsprinzip einer λ-Sonde.

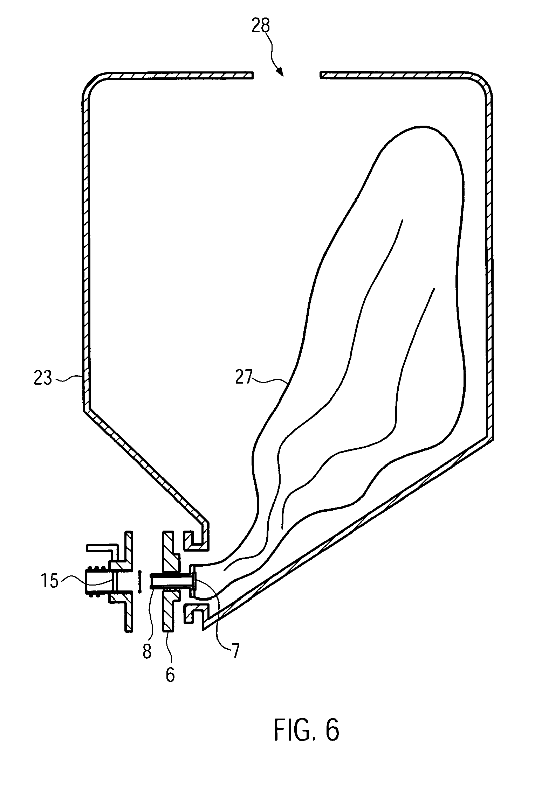

Fig. 6 zeigt schematisch einen Lebensmitteltank 23, hier ein Bag-in-Tank-System, zum Transport von Lebensmittelprodukten, wie beispielsweise Saft oder Fruchtzubereitung. Im Vergleich zu herkömmlichen Tanks haben die Bag-in-Tank-Systeme den Vorteil, dass nach der Benutzung der Tank nicht mehr aufwendig gereinigt und sterilisiert werden muss, da sich im Inneren des Tanks ein Inliner 27 befindet, der nach der Benutzung durch einen neuen ersetzt wird. Nach der Benutzung wird der Inliner 27 durch eine Öffnung 28 im Tank aus dem Inneren des Tanks entfernt und durch einen neuen Inliner ersetzt. Der neue Inliner wird so in den Tank 23 eingelegt, dass ein Auslaufstutzen 8 an einer entsprechenden unteren Öffnung im Tank herausschaut. Mit Hilfe einer Zentrierscheibe 6 kann der Auslaufstutzen 8 dann in der Öffnung zentriert werden und schließlich eine Ventileinrichtung 15 dichtend am Auslaufstutzen 8 bzw. am Zentrierring bzw. Anschlussflansch 6 befestigt werden. Um die Keimfreiheit des Inneren des Inliners 27 zu gewährleisten, ist üblicherweise noch eine Membran, Berstmembran oder Berstscheibe 7, am Auslaufstutzen 8 angeordnet. Diese Berst-membran 7 bedeckt den Ein- bzw. Auslaufkanal des Auslaufstutzens 8. Beim Einfüllen des Produktes reißt diese Membran fetzenfrei auf, so dass nur das Produkt ins Innere des Inliners gelangt.

Nachfolgend versteht man unter Anschlussbereich die Ventilvorrichtung 15 und den Auslaufstutzen 8 des Inliners bis zur Berstmembran 7. Die Ventilvorrichtung 15 kann beispielsweise mit Klemmschellen am Anschlussflansch 6 befestigt werden.

Die erläuterte Ausführungsform des Bag-in-Tank-Systems stellt hier nur ein Beispiel dar. Die Erfindung ist auch beispielsweise zum Sterilisieren von herkömmlichen Transportbehältern mit einem Anschlussbereich, beispielsweise einem Rohrstutzen, anwendbar.

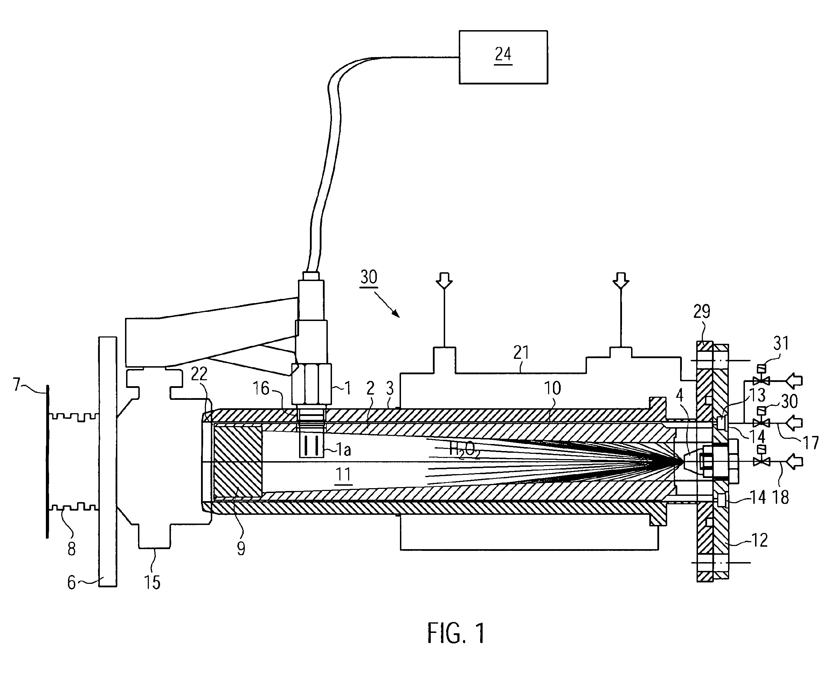

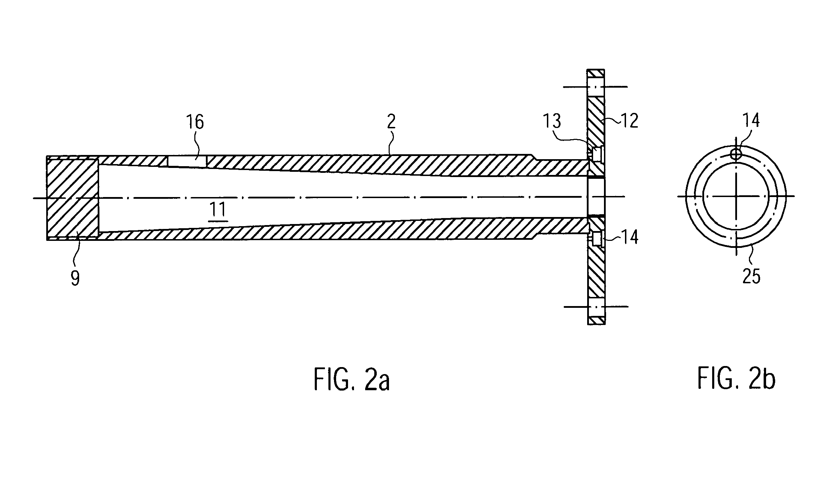

Fig. 1 zeigt eine Ausführungsform eines erfindungsgemäßen Sterilisationskopfes 30. Der Sterilisationskopf, wie er in Fig. 1 gezeigt ist, ist hier mit der Ventileinrichtung 15, also der Handklappe 15, zum Öffnen und Schließen des Kanals zur Membran 7, dichtend verbunden. Die Ventileinrichtung 15 ist hier z.B. am Anschlussflansch 6 befestigt und über diesen mit dem Beutelstutzen 8, der durch die Beutelmembran 7 verschlossen ist, verbunden. Wie insbesondere aus den Fig. 1 und 2 hervorgeht, umfasst der Sterilisationskopf 30 ein Innenrohr 2, dessen Durchmesser in einem Bereich von 20 bis 100 mm liegt. Der Durchmesser des Innenrohrs 2 nimmt dabei in Richtung Anschlussbereich zu, während die Außenabmessung, d.h. der äußere Durchmesser des Rohrs im Wesentlichen konstant bleibt. Am, von dem Anschlussbereich abgewandten, Ende des Innenrohrs 2 ist eine Einspritzdüse 4 vorgesehen, über die H₂O₂ in den Innenraum 11 des Innenrohrs 2 einspritzbar ist. Die Düse ist mit einer H₂O₂-Zuführungsleitung 18 verbunden. In der Leitung 18 ist ein Stellventil angeordnet. Somit kann H₂O₂ unter erhöhtem Druck, von etwa beispielsweise 5 bar, zugeführt werden. An dem, dem Anschlussbereich abgewandten, Ende des Innenrohrs 2 ist, wie insbesondere aus Fig. 2A hervorgeht, eine Ringscheibe 12 angeordnet, in die die Düse 4 eingesetzt ist und die einen Ringkanal 13 aufweist, der an der Rückseite der Scheibe 12 als erweiterte Ringnut endet. In diese Ringnut ist der in Fig. 2B gezeigte Abdeckring 25 eingesetzt, der eine Öffnung 14 aufweist, die mit dem Ringkanal 13 in Verbindung steht, und über die über eine Leitung 17 Dampf zuführbar ist.

Um das Innenrohr 2 ist im Wesentlichen konzentrisch das Außenrohr 3 angeordnet, wobei der Innendurchmesser des Außenrohrs 3 größer ist als der Außendurchmesser des Innenrohrs 2, so dass sich ein Ringkanal 10 zwischen Innenrohr 2 und Außenrohr 3 bildet. Dieser Ringkanal 3 ist zum Anschlussbereich hin als Ringspalt 22 geöffnet, so dass Dampf über den Ringspalt 22 in Richtung Anschlussbereich austreten kann. Der Ringkanal 10 ist darüber hinaus hier über den Ringkanal 13 in der Abdeckscheibe 12 mit der Dampfzuleitung 17 verbunden. Somit kann Dampf über die Zuleitung 17 und den Ringkanal 10 dem Anschlussbereich und somit auch dem Beutelstutzen und der Beutelmembran zugeführt werden. Das Außenrohr weist beispielsweise einen Innendurchmesser von 20 bis 100 mm und einen Außendurchmesser von 20 bis 100 mm . Wie aus Fig. 1 hervorgeht, ist das Außenrohr 3 an seinem, dem Anschlussbereich abgewandten Ende, hier über einen Anschlussflansch 29 mit der Scheibe 12 verbunden.

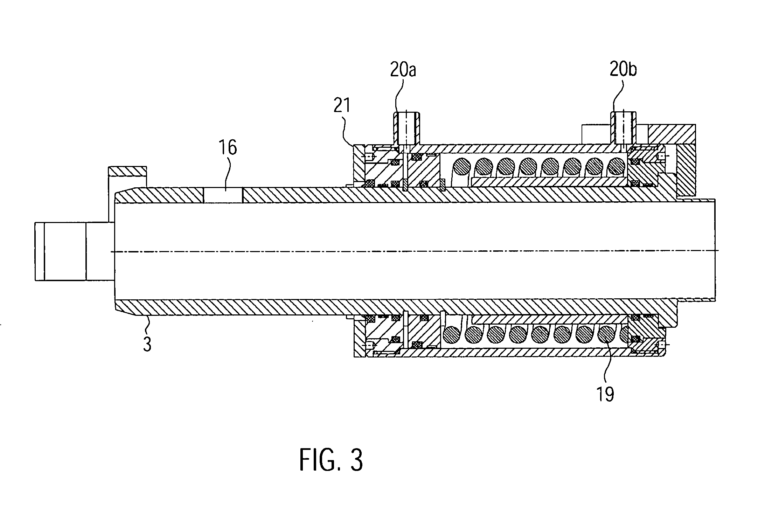

Wie insbesondere aus Fig. 3 hervorgeht, umfasst hier das Außenrohr 3 eine Andockeinrichtung 21, die das Außenrohr 3 zusammen mit dem Innenrohr 2 in Richtung Anschlussbereich, d.h. hier zu der Ventileinrichtung 15 drückt, damit der Sterilisationskopf 30 druckdicht mit dem Anschlussbereich verbindbar ist. Dazu weist die Andockeinrichtung 21 eine pneumatisch betätigbare Druckfeder 19 auf, sowie entsprechende Anschlüsse 20a, b für Prozessluft. In dem Sterilisationskopf 30, d.h. im Innenraum 11, in den das H₂O₂ eingespritzt, bzw. eingesprüht wird, hier dem Innenrohr 2, ist der Sensorkopf 1 a des Sensors 1 angeordnet. Dazu weist der Sterilisationskopf, hier das Innenrohr 2 und das Außenrohr 3, eine entsprechende Öffnung 16 auf. Zwischen Sterilisationskopf und Öffnung 16 ist eine entsprechende Dichtung vorgesehen.

Der Sensor 1 dient hier zur Messung der Sauerstoffkonzentration im Innenraum 11. Vorteilhafterweise handelt es sich hier um einen Sensor 1, der nach dem Prinzip einer λ-Sonde arbeitet. Dabei gibt es im Wesentlichen zwei Messprinzipien. Zum Einen kann die sauerstoffabhängige Spannungsänderung an einem Festkörperelektrolyten bestimmt werden (Nernstsonde). Auch die Messung der Widerstandsänderung einer Keramik (Widerstandssonde) ist möglich.

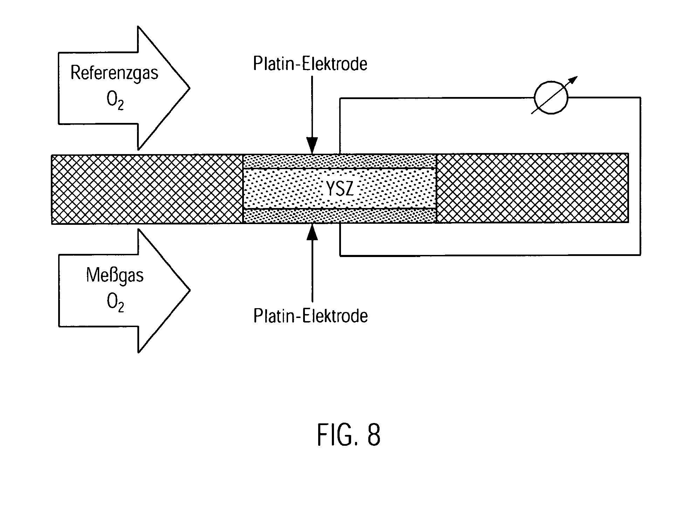

Fig. 8 zeigt ein Funktionsprinzip der λ-Sonde. Die λ-Sonde weist einen Festkörperelektrolyten, hier in Form von einer Yttrium dotierten Zirkoniumdioxid-Keramik auf. Die Oberfläche der Keramik ist auf zwei Seiten mit porösem, also gasdurchlässigem, stromleitfähigem Platin beschichtet. Auf der einen Seite der keramischen Sonde ist Referenzgas mit einem bestimmten Sauerstoffgehalt, z.B. Luft, angeordnet, während die andere Seite der Keramik mit dem Messgas in Verbindung steht. Die Sonde wird auf etwa über 300 °C geheizt. Bei Temperaturen über etwa 300 °C wird die Yttrium dotierte Zirkoniumdioxid-Keramik der Sonde für negative Sauerstoff-Ionen leitend. Die Sauerstoffatome können als doppelt negativ geladene Ionen durch die Keramik durchtreten. Die zur Ionisierung der Sauerstoffatome erforderlichen Elektronen werden von den leitfähigen Elektroden geliefert. Dadurch lässt sich zwischen den beiden angeordneten Elektroden eine elektrische Spannung, die Sondenspannung, abnehmen, die vom Sauerstoffgehalt abhängt.

Möglich ist auch der Einsatz einer Widerstandssonde. Hier besteht das Sensorelement beispielsweise aus einer halbleitenden Keramik, beispielsweise Titandioxidkeramik. Die Ladungsträger werden durch Sauerstoff-Fehlstellen, die als Donatoren wirken, zur Verfügung gestellt. Bei umgebendem Sauerstoff werden die Fehlstellen besetzt und reduzieren die Zahl der freien Ladungsträger. Die Sauerstoff-Ionen tragen hier nicht wesentlich zur Leitfähigkeit bei, sondern der Sauerstoff reduziert die Zahl der freien Ladungsträger. Bei hoher Sauerstoffkonzentration hat das Sensormaterial einen großen Widerstand. Das Messsignal wird z.B. durch einen Spannungsteiler mit einem festen Widerstand erzeugt.

Da sich bei der Behandlung mit Dampf, der Sterilisationskopf, und demnach auch die Innenwände des Sterilisationskopfs, hier die Innenwände des Innenrohrs 2, bis zu etwa 130 °C aufheizen, führt die Wärme im Sterilisationskopf 30 dazu, dass folgende Reaktion auftritt: 2H₂O₂ → 2H₂O + O₂.

Der Sauerstoff kann wie zuvor beschrieben über die Sonde 1 erfasst werden, so dass eine Aussage gemacht werden kann, ob eine Sterilisation erfolgt ist oder nicht. Bereits wenige Sekunden nach dem Einspritzen des H₂O₂ durch die Einspritzdüse 4, wird ein entsprechender Peak der Sauerstoffkonzentration erfasst. Das Messsignal wird über eine entsprechende Leitung einer Auswerteeinheit 24 zugeführt. Die Auswerteeinheit 24 vergleicht die gemessene Sauerstoffkonzentration mit einem Schwellwert und bestimmt bei Überschreiten des Schwellwerts innerhalb eines bestimmten Zeitraums, dass eine ausreichende Sterilisation mittels H₂O₂ stattgefunden hat. Dabei werden Werte in einem Zeitraum von 1 bis 10 Sekunden nach dem Ausstoßen des H₂O₂ herangezogen, und nicht Werte die später auftreten, da, auch wenn kein Wasserstoffperoxyd eingespritzt wird, sich die Sauerstoffkonzentration in dem Raum 11 langsam bis auf 21 % erhöhen könnte. Eine erhöhte Sauerstoffkonzentration ist jedoch, gemäß der vorliegenden Erfindung, bereits nach 2 bis 4 Sekunden in Form eines Peaks nach dem Einspritzen des H₂O₂ detektierbar.

Bei der Sterilisation mit Wasserstoffperoxyd dient die Zuleitung 10 für Dampf als Entlüftungsleitung um einen Überdruck im Anschlussbereich abzubauen. Dabei wird das Ventil 30 zur Dampfversorgung geschlossen und ein Abluftventil 31 geöffnet. Es kann jedoch auch ein separates Dampfrohr als Entlüftungsrohr vorgesehen sein, oder aber ein zusätzliches Entlüftungsrohr.

Vorteilhafterweise befindet sich zwischen der Einspritzdüse 4 und dem Ende der Entlüftungsleitung, hier dem zum Anschlussbereich gewandten Ringspalt 22, eine Dampfbarriere. Die Dampfbarriere bremst das Abströmen von H₂O und O₂ beim Zerfall von H₂O₂. Dies ist vorteilhaft, da bei heißen Wänden des Messkopfes das Wasser sofort verdampfen und zusammen mit dem Sauerstoff über die Entlüftungsleitung, hier den Ringkanal 10, entweichen würde. Damit würde auch der Sauerstoff verdrängt werden, was eine Messung des Wasserstoffperoxyds über den Sauerstoff schwieriger machen würde, da der Sensor dann weit sensibler messen müsste. Durch die Dampfbarriere 9 wird jedoch das Abströmen von Wasserdampf und Sauerstoff gebremst. Somit entsteht in dem Innenraum 11 ein leichter Überdruck von 10 bis 100 mbar . Der Sauerstoff wird somit nicht so schnell durch den Wasserdampf verdrängt, so dass sich eine erhöhte Sauerstoffkonzentration im Innenbereich 11 zwischen Dampfbarriere 9 und Einspritzdüse 4 ergibt.

Die Dampfbarriere 9 ist dabei vorzugsweise aus einer Packung gebildet, die in das Innenrohr 2 eingepasst ist. Dabei weist die Innenfläche des Innenrohrs 2 eine entsprechende Aussparung auf, in die die Packung 9 eingesetzt ist. Die Packung weist dabei eine Vielzahl von Durchgangskanälen auf, die einen Druckabfall des durchströmenden Mediums bewirken. Eine solche Packung kann beispielsweise eine Packung sein wie sie für Laborkolonnen verwendet werden (z.B. Sulzerpackung). Die Packungen können beispielsweise aus Metallblech gebildet sein, oder aber aus einer grobporösen Keramik. Auch der Einsatz von Stahlwolle etc. ist denkbar.

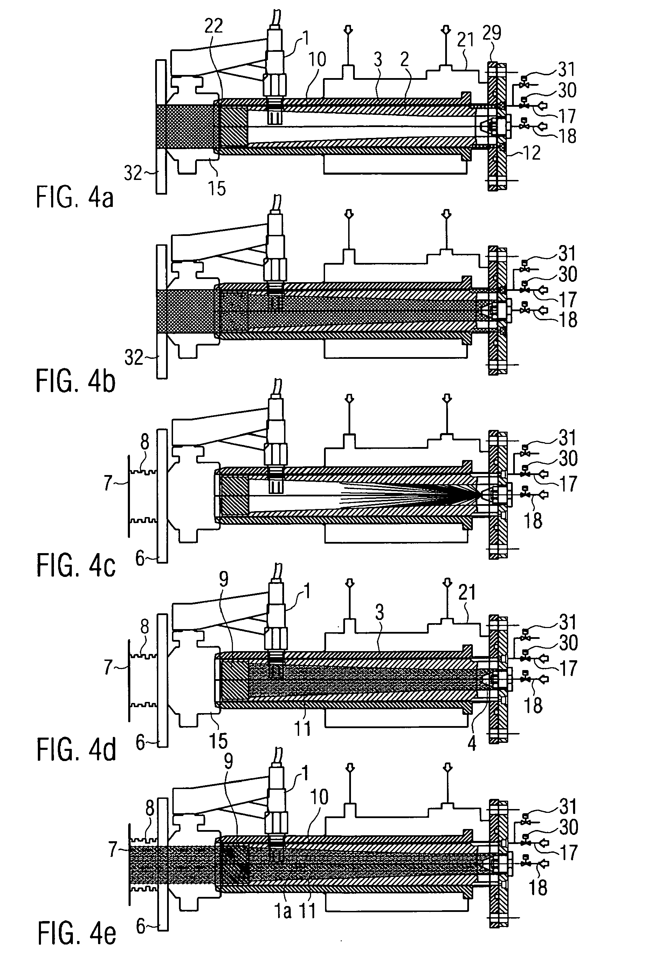

Das erfindungsgemäße Verfahren wird nachfolgend in Zusammenhang mit den Figuren 4 bis 5 und 7 näher erläutert.

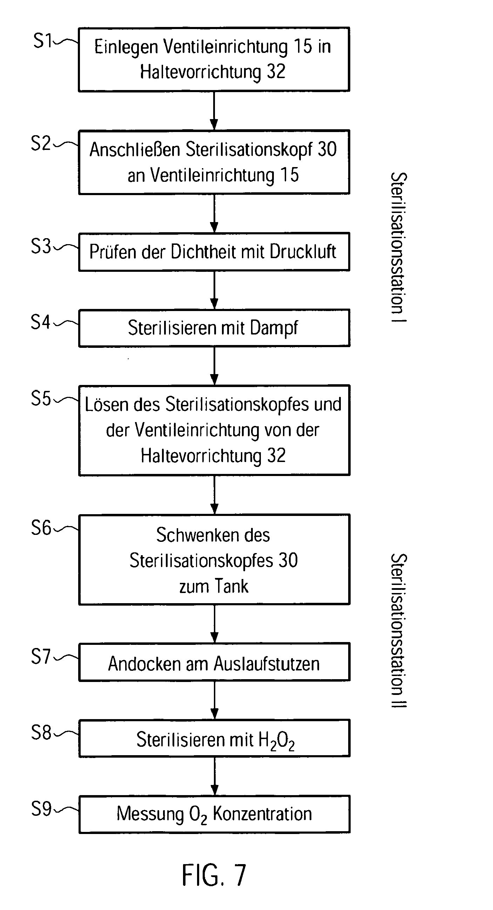

Zunächst wird wie aus Fig. 4a und Schritt S1 in Fig. 7 hervorgeht, die von dem Tank entkoppelte Ventileinrichtung 15 in eine Haltevorrichtung 32 eingelegt. Der Sterilisationskopf 30 wird dann an die Ventileinrichtung 15 mit Hilfe der Andockeinrichtung 21 druckdicht angeschlossen (S2). Es kann in Schritt S3 eine Prüfung der Dichtheit mit Druckluft erfolgen. Schließlich erfolgt eine Sterilisation der geöffneten Ventileinrichtung, hier der Handklappe 15, mit Dampf (S4), wobei Dampf über die Dampfzuleitung 17, bei geöffnetem Ventil 30, dem Ringkanal 10 zugeführt wird und über den Ringspalt 22 der Ventileinrichtung 15 zugeführt wird (siehe auch Fig. 4a).

Hier erfolgt die Sterilisation der Ventileinrichtung in nicht eingebautem Zustand. Die vorliegende Erfindung soll jedoch nicht darauf beschränkt sein, die Sterilisation mit Dampf kann ebenfalls in einem Zustand erfolgen in dem die Ventileinrichtung 15 bereits am Transportbehälter angeordnet ist. Der Dampf ist hier durch die grau hinterlegten Abschnitte dargestellt. Wie aus Fig. 4b hervorgeht, erfolgt nach der Dampfströmung zwischen Innen- und Außenrohr ein Einströmen des Dampfes in das Innenrohr. Durch die Sterilisation mit Dampf wird das Innenrohr 2 bis auf etwa 130 °C aufgeheizt.

Schließlich wird der Sterilisationskopf 30 zusammen mit der Ventileinrichtung von der Haltevorrichtung 32 gelöst (siehe Schritt S5) und wird zum Tank hin geschwenkt (Schritt S6). Die Schwenkung kann dabei über einen nicht dargestellten beweglichen Arm erfolgen, der mit dem Sterilisationskopf verbunden ist. Der Sterilisationskopf 30 wird nun druckdicht mit dem Anschlussbereich, d.h. dem Innenraum des Beutelstutzens 8, sowie dem Innenraum des Ventils 15, verbunden (S7). Die Ventileinrichtung 15 wird dabei an dem Anschlussflansch 6 befestigt. Wie insbesondere aus Fig. 4c hervorgeht, wird nun über die Einspritzdüse 4 unter einem Überdruck von z.B. etwa 5 bar, H₂O₂ bei geöffnetem Ventil in der Leitung 18 dem Innenraum 11 zugeführt. Dabei werden beispielsweise insgesamt 1 bis 3 g H₂O₂ über z.B. 3 s eingesprüht. (Siehe auch Schritt S8). Wie durch die grau hinterlegten Abschnitte gekennzeichnet, breitet sich das H₂O₂ im Innenraum 11 aus.

Wie aus Fig. 4e hervorgeht, verteilt sich das H₂O₂ durch die Dampfbarriere hindurch, durch den Kanal in der Ventileinrichtung 15 bis in den Beutelstutzen 8. Die Membran 7 des Inliners ist die kälteste Stelle, so dass es hier zu einer Kondensation des Wasserstoffperoxyds und des Wasserdampfs kommt. Somit erfolgt in diesem Bereich eine ausreichende Sterilisierung. Der Ringkanal 10 kann dann einen Überdruck abbauen, dient hier also als Entlüftungsleitung bei geöffnetem Ventil 31 und geschlossenem Ventil 30. Da, wie insbesondere in Fig. 4d gezeigt, die Wandtemperatur z.B. zwischen 130 und 120 °C liegt, zerfällt Wasserstoffperoxyd in Wasser- und Sauerstoff. Durch die Dampfbarriere 9 wird der Sauerstoff nicht gleich durch den Wasserdampf über den Ringspalt 10 abgeleitet. Es ergibt sich in Raum 11 eine erhöhte Sauerstoffkonzentration, die über den Sensor 1 gemessen wird (S9). Die Messwerte werden an die Auswerteeinheit 24 weitergeleitet.

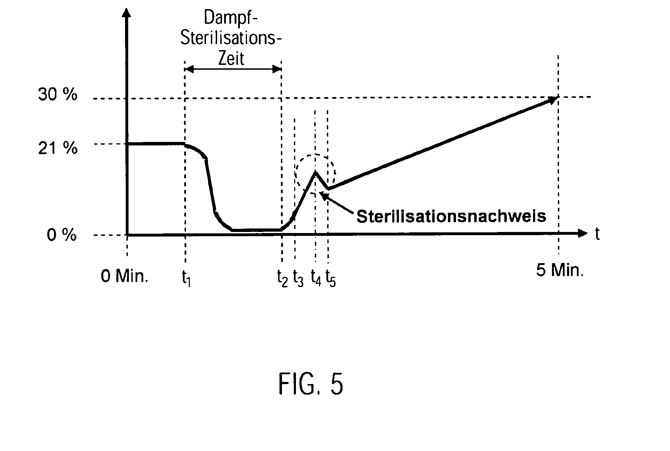

Wie insbesondere aus Fig. 5 hervorgeht, misst der Sensor beispielsweise bei Raumluft in dem Sterilisationskopf, hier im Innenraum 11, eine Konzentration von 21 %. Zu einem Zeitpunkt t1 erfolgt die Dämpfung mit H₂O₂ (siehe auch Schritt S4 bzw. Fig. 4a, b). Durch den Wasserdampf im Raum 11 sinkt der Messwert beim Dämpfen auf nahezu 0 ab. Nach dem Dämpfen, d.h. nach dem Zeitpunkt t2 wird der Messkopf 30 von der Halterung 32 abgekoppelt und am Tank angedockt (Schritt S7). Zum Zeitpunkt t3 erfolgt dann das Einsprühen, bzw. Einspritzen des Wasserstoffperoxyds. Wie deutlich aus Fig. 5 hervorgeht, erfolgt (durch das Vergasen und den Zerfall des H₂O₂) unmittelbar danach zum Zeitpunkt t4 ein Peak, der eine erhöhte Sauerstoffkonzentration anzeigt. Ein solcher Peak, d.h. ein solcher Spitzenwert wird mit einem Schwellwert verglichen, wobei bei Überschreiten des Schwellwerts bestimmt wird, dass eine ordnungsgemäße Sterilisation mit Wasserstoffperoxyd stattgefunden hat. Der Peak tritt in einem Zeitraum von 1 bis 10 s auf und wird in diesem Zeitraum zur Auswertung herangezogen. Der Spitzenwert, der von 1 bis 10 s nach Einspritzen des Wasserstoffperoxyds detektiert wird, steigt von 0% in einen Bereich von etwa 15 bis 18 % Sauerstoff. Das H₂O₂ Gas expandiert und strömt dabei zur Membran 7. Dadurch nimmt die Konzentration im Raum 11 bis zu einem Zeitpunkt t5 ab. Anschließend steigt die Sauerstoffkonzentration deutlich über Atmosphärenkonzentration von 21% durch die Kondensation des Wasserdampfanteils beim Abkühlen. Nach 3 bis 6 min kann ein Wert von > 21% bis 50% gemessen werden.

Bei dem vorherigen Ausführungsbeispiel wird vor der Sterilisation mit Wasserstoffperoxyd eine Dampfsterilisation durchgeführt, so dass sich die Innenwände des Sterilisationskopfes aufheizen, was wiederum einen Zerfall des H₂O₂ beschleunigt. Sollte keine solche Dampfsterilisation erwünscht sein, könnten die Innenwände des Sterilisationskopfs 30, hier die Innenwände des Innenrohrs 2, auch über eine Heizeinrichtung aufgeheizt werden.

Die vorliegenden Ausführungsbeispiele wurden im Zusammenhang mit der Sterilisation eines Anschlussbereiches eines Lebensmitteltanks beschrieben. Grundsätzlich kann jedoch bei jeder Sterilisation mit H₂O₂ über die Messung der Sauerstoffkonzentration in einem Raum, in dem H₂O₂ eingebracht wird, bestimmt werden, ob eine Sterilisation stattgefunden hat oder nicht. Dabei kann der, wie in Zusammenhang mit dem vorherigen Ausführungsbeispiel gezeigte Sensor 1 verwendet werden. Gegebenenfalls muss dann der Raum in dem das H₂O₂ eingeleitet wird, zumindest teilweise aufgeheizt werden, so dass der Raum Wandtemperaturen in einem Bereich von 90 bis 130 Grad aufweist. Somit kann auf einfache Art und Weise das Vorhandensein von H₂O₂ durch Anstieg der Sauerstoffkonzentration bestimmt werden und auch zuverlässig dokumentiert werden.

## Patentansprüche

1. Vorrichtung zum Sterilisieren, insbesondere zum Sterilisieren eines Anschlussbereichs (15, 8, 7) eines Lebensmitteltanks (23) mit einem Sterilisationskopf (30), in dem eine Einspritzdüse (4) für H₂O₂ angeordnet ist,
**dadurch gekennzeichnet, dass** in dem Sterilisationskopf (30) ein Sensor (1) angeordnet ist, der die Sauerstoffkonzentration in dem Sterilisationskopf (30) erfassen kann.

2. Vorrichtung nach Anspruch 1,
**dadurch gekennzeichnet, dass** der Sensor (1) nach dem Prinzip einer λ-Sonde die Sauerstoffkonzentration misst.

3. Vorrichtung nach Anspruch 1 oder 2,
**dadurch gekennzeichnet, dass** der Sensor einen Nernstsonde oder eine Widerstandssonde umfasst.

4. Vorrichtung nach mindestens einem der Ansprüche 1 bis 3,
**dadurch gekennzeichnet, dass** die Vorrichtung eine Auswerteeinheit umfasst, die die gemessene Sauerstoffkonzentration mit einem Schwellwert vergleicht und bei Überschreiten des Schwellwerts bestimmt, dass eine ausreichende Sterilisation mittels H₂O₂ stattgefunden hat.

5. Vorrichtung nach mindestens einem der Ansprüche 1 bis 4,
**dadurch gekennzeichnet, dass** im Sterilisationskopf (30) weiter eine Entlüftungsleitung (10) vorgesehen ist, und zwischen Entlüftungsleitung (10) und der Einspritzdüse (4) eine Dampfbarriere (9) angeordnet ist, die das Abströmen von H₂O und O₂ bei der H₂O₂-Sterilisation bremst, wobei der Sensor (1) in einem Raum (11) zwischen Dampfbarriere (9) und Einspritzdüse (4) angeordnet ist.

6. Vorrichtung nach Anspruch 5,
**dadurch gekennzeichnet, dass** im Sterilisationskopf (30) weiter eine Zuleitung (10) für Dampf vorgesehen ist, die bei der Sterilisation mit H₂O₂ als die Entlüftungsleitung dient.

7. Vorrichtung nach Anspruch 5 oder 6,
**dadurch gekennzeichnet, dass** der Sterilisationskopf (30) ein Innenrohr (2) umfasst, sowie ein darüber angeordnetes Außenrohr (3), wobei zwischen Innen- und Außenrohr ein Ringkanal (10) vorgesehen ist, der die Zuleitung für Dampf (10) darstellt, wobei die Einspritzdüse (4) in dem Innenrohr (2) angeordnet ist.

8. Vorrichtung nach mindestens einem der Ansprüche 5 bis 7,
**dadurch gekennzeichnet, dass** die Dampfbarriere (9) aus einer Packung mit einer Vielzahl von Durchgangskanälen gebildet ist, die einen Druckabfall des durchströmenden Mediums bewirkt.

9. Verfahren, insbesondere zum Sterilisieren eines Anschlussbereichs eines Lebensmitteltanks mit einer Vorrichtung nach mindestens einem der Ansprüche 1 bis 8 mit folgenden Schritten:
Anschließen des Sterilisationskopfes (30) an einen Anschlussbereich (15, 8, 7), Einspritzen von H₂O₂ aus einer Einspritzdüse (4) in dem Sterilisationskopf (30) in Richtung Anschlussbereich
**gekennzeichnet durch**
Messen der Sauerstoffkonzentration im Sterilisationskopf (30) nachdem H₂O₂ ausgestoßen wurde und
Bestimmen ob eine Sterilisation stattgefunden hat in Abhängigkeit der gemessenen Sauerstoffkonzentration.

10. Verfahren nach Anspruch 9,
**dadurch gekennzeichnet, dass** vor dem Einspritzen von H₂O₂ vom Sterilisationskopf (30) über eine Zuleitung (10) heißer Wasserdampf ausgestoßen wird.

11. Verfahren nach Anspruch 9 oder 10,
**dadurch gekennzeichnet, dass** die Temperatur im Messkopf beim Ausstoßen des H₂O₂ zwischen 90 und 130 Grad liegt.

12. Verfahren nach mindestens einem der Ansprüche 9 bis 11,
**dadurch gekennzeichnet, dass** die innerhalb eines bestimmten Zeitpunkts nach dem Einspritzen des H₂O₂ gemessene Sauerstoffkonzentration mit einem Schwellwert verglichen wird und bei Überschreiten des Schwellwerts bestimmt wird, dass eine ausreichende Sterilisation mittels H₂O₂ stattgefunden hat.

13. Verfahren nach mindestens einem der Ansprüche 10 bis 12,
**dadurch gekennzeichnet, dass** bei der Sterilisation mit H₂O₂ eine Entlüftungsleitung (10) vorgesehen ist, wobei zwischen der Einspritzdüse und der Entlüftungsleitung (10) eine Dampfbarriere (9) angeordnet ist, die das Ausströmen von H₂O₂ und O₂ bremst.

14. Verfahren nach Anspruch 13,
**dadurch gekennzeichnet, dass** zwischen Dampfbarriere (9) und Einspritzdüse ein Druck von 10 bis 100 mbar aufgebaut wird nachdem H₂O₂ ausgestoßen wurde.

15. Verfahren nach mindestens einem der Ansprüche 9 bis 13,
**dadurch gekennzeichnet, dass** in einem Zeitraum von 1 bis 10 s nach dem Ausstoßen des H₂O₂ eine erhöhte Sauerstoffkonzentration erfasst wird.

16. Verfahren zum Bestimmen von H₂O₂, insbesondere zum Bestimmen, ob eine Sterilisation mit H₂O₂ erfolgt ist,
**dadurch gekennzeichnet, dass** die Sauerstoffkonzentration in einem Raum, in dem H₂O₂ eingebracht wird, mit Hilfe eines Sensors ermittelt wird.
